(19) **Europäisches Patentamt**

**European Patent Office**

**Office européen des brevets**

(11) Publication number: **0 284 999 B1**

(12) **EUROPEAN PATENT SPECIFICATION**

(45) Date of publication of patent specification: **11.12.91**  (51) Int. Cl.⁵: **A61K 47/00, A61K 33/18**

(21) Application number: **88104709.6**

(22) Date of filing: **24.03.88**

(54) **Pharmaceutical preparation containing iodine.**

(30) Priority: **02.04.87 DE 3711083**

(43) Date of publication of application:
**05.10.88 Bulletin 88/40**

(45) Publication of the grant of the patent:
**11.12.91 Bulletin 91/50**

(84) Designated Contracting States:
**AT BE CH DE ES FR GB GR IT LI NL SE**

(56) References cited:
**EP-A- 0 185 490
DE-C- 389 778
GB-A- 414 098
US-A- 4 012 504
US-A- 4 301 145**

**HELWIG "Moderne Arzneimittel", 1980, page
585**

(73) Proprietor: **Euro-Celtique S.A.
122 Boulevard de la Petrusse
Luxemburg(LU)**

(72) Inventor: **Rackur, Helmut, Dr.
Siemensstrasse 2
W-6250 Limburg / Lahn(DE)**
Inventor: **Pinter, Erwig, Dr.
Höhenweg 25
W-3250 Hameln(DE)**

(74) Representative: **Eisenführ, Speiser & Strasse
Martinistrasse 24
W-2800 Bremen 1(DE)**

## Description

The invention concerns a pharmaceutical, preparation containing a solvent and polyvinylpyrrolidone-complexed iodine, as well as the use of such a preparation.

Solutions of iodine in organic solvents with a considerable water content have been used for a long time, e.g. in the form of the well-known tincture of iodine, in pharmaceutical applications for disinfection.

These known, highly aqueous iodine solutions are still to be improved for some application purposes. If, for example, a patient is to be antiseptically treated in a specific skin area in preparation for an operation, a vaccination, for example, prior to applying a cover which leaves the area to be operated upon open, then considerable problems arise from the relatively slow evaporation of the solvent, which is due to the especially high heat of evaporation of water. On one hand, the patient and the operating surgeon have to accept rather long time intervals until evaporation of the solvent has progressed sufficiently; on the other hand, evaporating the highly aqueous solvent requires a large amount of heat which must be provided by the patient's body in the operating area. The patient usually experiences this marked heat loss as irritation.

Furthermore, iodine precipitates from the known solutions in the form of crystalline particles when the solvent is evaporated, and these particles can lead to an inhomogeneous iodine application on the body area to be disinfected sometimes leads to marked skin irritation. These skin irritations result especially in the case of high local iodine concentrations (compare K.H. Wallhäußer: Sterilisation, Desinfektion, Konservierung, 2nd edition, Thieme 1978, page 345).

Moreover, the aqueous solutions cannot be stored over long time periods without loss of activity. This is valid for complexed as well as non-complexed iodine preparations, even though specific aqueous preparations with polyvinylpyrrolidone-complexed iodine show rather long shelf life.

Furthermore, the known highly aqueous iodine solutions are suitable for killing bacterial spores only after action times of about 30 seconds and more, and therefore are only of limited use for fast, reliable disinfection.

As is well known, lower alcohols with considerable water content (usually 30% - 50%) exhibit microbicidal activity due to protein denaturation; with low water content (about 5%), ethanol and propanol have only a conserving effect on many germs and the spores are not killed. Ethanol with little or no water content is even used for storage conservation of specific germs (e.g. anthrax).

Therefore, 70% ethanol or 50-70% n- or i-propanol are usually used for disinfection of hands and skin (compare Wallhäußer: loc.cit., pp. 391-394).

However, even for most aqueous alcohols, application times of up to 30 seconds duration are prescribed by corresponding regulations, which is impractical for many of purposes (e.g. preparing a vaccination) and sporicidal effects are not ensured by this.

From GB-A-414 098, pharmaceutical preparations containing colloidal iodine in petrolatum, glycerol or glycol are known, which are prepared by dissolving iodine vapour in the heated solvent. These preparations are not very stable and are further restricted to solvents which do not react with iodine vapour even when heated.

From US-A-4,012,504, iodine is dissolved in heated mineral oil in order to prepare a solution for preventing bovine mastites. According to this prepublication, alcohol should not be present in the composition, even at very low concentrations of about 5%. Instead, the solutions contain about 5% water.

US-A-4,301,145 discloses an antiseptic skin cream, containing, besides petrolatum, cetyl alcohol and stearyl alcohol, glycerine, sodiumlauryl sulphate and polyvinylpyrrolidone iodine.

It is therefore an object of the invention to provide a pharmaceutical preparation of the aforementioned kind with an especially long shelf life, which evaporates with minimal irritation of the patient treated therewith; which ensures a homogeneous distribution of iodine in the application area; and which gives a faster, reliable microbicidal and also a sporicidal effect.

In order to accomplish this object, the pharmaceutical preparation is, according to the invention, provided with the features defined in the characterizing part of patent claim 1.

Advantageous embodiments are defined in subclaims 2-6; advantageous uses of the preparation defined in the above-mentioned claims are described in claims 7-9.

Initially, there were misgivings when considering pharmaceutical preparations according to the invention, the water contents of which are substantially lower than those of known preparations. On the one hand, increased irritation due to local iodine excesses was to be reckoned with in case of any marked water content reduction; on the other hand it was to be expected that patients would feel uncomfortable due to the much faster and therefore stronger cooling in the evaporation area even in view of the smaller overall heat loss.

It was further to be expected that the relatively fast microbicidal action of alcoholic solvents would be completely lost when reducing water content, since it has always been maintained that a substantial water content is indispensible.

The advantages of the invention therefore comprise the facts, that the preparation according to the invention can be stored without deterioration for a very long time (more than two years) when not diluted with water, and that the preparation after application evaporates fast and therefore without any undesirable delay of the intended operation. The preparation is preferably used undiluted for antiseptic preparation of operations on living beings and shows the previously mentioned advantageous fast evaporation accompanied by immediate microbicidal and virucidal effect. Thus, for instance a vaccination can be carried out practically without any delay. When using iodine complexed with polyvinylpyrrolidone (PVP), an even, highly adherent film is formed on the skin, which is especially advantageous; the formation of any irritating crystalline iodine particles is prevented thereby to a high degree. For the disinfection of rooms and objects such as operation apparatus, and surgical instruments, the solution can be diluted with water if desired. This makes the use of the preparation more economical in cases when fast evaporation is not required.

It has unexpectedly been found, that the non-diluted preparation is also well tolerated by the skin and is not felt to be irritating by patients.

A special advantage of the preparation according to the invention lies in its unexpected effectiveness in killing bacterial spores. Even after a short action time in the range of some 10 seconds, which was up to now not obtainable, bacterial spores are killed to a high degree. Thereby the antiseptic effect obtainable through use of the preparation is largely increased in comparison with known preparations.

Based on efficiency studies carried out with different diluted samples of the preparation according to the invention as well as with different diluted alcohols and aqueous (complexed) iodine solutions, it can be concluded that a synergistic coaction of solvent and iodine is present in the invention's preparations, since the preparation even in its non-aqueous state has a practically immediate microbicidal effect. At relatively high water content (e.g. after dilution), the preparation according to the invention shows a high microbicidal effect even within 10 seconds after application when the invention's mixture of alcohols is used as the preparation's solvent. This 10-second effect is even present when the preparation is diluted with water up to a ratio of 1:3. While this effect could initially be considered to derive from the well-known effect of aqueous alcohol, it is apparently due to said synergistic coaction, since the non-aqueous preparation shows the same 10-second effect.

No reaction of the iodine with the organic solvent is ever noted. This is not only pertinent for the shelf life of the preparation. A reaction of isopropanol with iodine could lead to the production of, iodoacetone which is a strong lachrymator and would strongly impair the usefulness of the preparation. Yet even after storing the preparation for several years, no iodoacetone formation is observed.

Hereafter, an especially preferred embodiment of the invention will be discussed in greater detail and in comparison with reference preparations.

A pharmaceutical preparation according to the invention was prepared by mixing ethanol, i-propanol and iodine complexed with polyvinylpyrrolidone. This test preparation contained 48% ethanol, 48% i-propanol and 4% PVP-iodine; the test preparation was non-aqueous.

As laid down in the pertinent regulations of the DGHM ("Deutsche Gesellschaft für Hygiene und Mikrobiologie"), germicide kinetics were determined in a quantitative suspension experiment with staphylococcus aureus using the test preparation as the germicide. Inactivation of the germicide was effected in respective samples after 10, 15, 20, 25, 30 seconds; 1, 2 and 5 minutes, respectively, by addition of a mixture of 3% Tween® 80, 0.3% lecithine, 0.5% sodium thiosulfate and 0.1% histidine to the respective sample.

The results showed that within 10 seconds, practically quantitative germicidal action (reduction factor at least 5) had been achieved.

Also according to the "Regulations for examination and evaluation of chemical disinfection methods" of the DGHM, tests for killing
Staphylococcus aureus ATCC 6538;
Escherichia coli ATCC 11229;
Pseudomonas aeruginosa ATCC 15442;
Proteus mirabilis ATCC 14153 and
Candida albicans ATCC 10231
were carried out. The inactivation of the germicide test preparation was carried out as described above. As a reference, aqueous i-propanol (60% by volume) was used.

A stronger effect of the preparation according to the invention as compared to the reference (mean value of log Reduction Factor) was found after 30 seconds under the conditions of DGHM regulation II.1 "hygenic disinfection of hands", and after 5 minutes when tested according to regulation II.2 "surgical disinfection of hands".

Corresponding dilution experiments with the preparation according to the invention showed a lower limit concentration for growth inhibition (regulation I.2.1) of 2.5% for all above-mentioned germs.

The 30-seconds germicidal effect (regulation I.2.2) was found to comply with the regulation for all above-mentioned germs even when the preparation was diluted with water to the following extent:

E. coli: 0.5 %;

P. mirabilis: 1.0 %;

P. aeruginosa: 2.5 %;

S. aureus: 2.5 %;

C. albicans: 0.5 %.

Moreover, in the presence of albumin (regulation I.2.3.1 and 2.3.2) a reduction factor of 5 resulted for S. aureus and P. aeruginosa within 30 seconds, when the non-aqueous preparation was used.

## Claims
### Claims for the following Contracting States : DE, GB, FR, IT, NL, SE, LI, CH, BE, AT

1. A pharmaceutical preparation containing a solvent and polyvinylpyrrolidone-complexed iodine, characterized in that the solvent is a mixture of ethanol and isopropanol in a ratio between 2:1 and 1:2, the preparation containing not more than 6% by weight of water.

2. Preparation according to Claim 1, characterized in that the solvent contains ethanol and isopropanol in a 1:1 ratio.

3. Preparation according to Claim 1 or 2, characterized in that the preparation does not contain more than 2% by weight and especially between 0 and 0.5% by weight of water.

4. Preparation according to any one of Claims 1 to 3, characterized in that the preparation contains polyvinylpyrrolidone in an amount sufficient for completely complexing the $I_2$-content.

5. Preparation according to any one of Claims 1 to 4, characterized in that the $I_2$-content of the preparation lies between 0.05 and 2% by weight.

6. Preparation according to Claim 5, characterized in that the $I_2$-content lies between 0.1 and 1% by weight.

7. Use of the preparation according to any one of Claims 1 to 6 for the external disinfection of living beings.

8. Use of the preparation according to any one of Claims 1 to 6 for the disinfection of rooms and/or objects.

9. Use of the preparation according to any one of Claims 1 to 6 for killing bacterial spores.

### Claims for the following Contracting States : ES, GR

1. A method for producing a pharmaceutical preparation containing a solvent and polyvinylpyrrolidone-complexed iodine, characterized in that as the solvent, a mixture of ethanol and isopropanol in a ratio between 2:1 and 1:2 is used and the preparation does not contain more than 6% by weight of water.

2. Method according to Claim 1, characterized in that the solvent contains ethanol and isopropanol in a 1:1 ratio.

3. Method according to Claim 1 or 2, characterized in that the preparation does not contain more than 2% by weight and especially between 0 and 0.5% by weight of water.

4. Method according to any one of Claims 1 to 3 characterized in that the preparation contains polyvinylpyrrolidone in an amount sufficient for completely complexing the $I_2$-content.

5. Method according to any one of Claims 1 to 4, characterized in that the $I_2$-content of the preparation lies between 0.05 and 2% by weight.

6. Method according to Claim 5, characterized in that the $I_2$-content lies between 0.1 and 1% by weight.

7. Use of the preparation according to any one of Claims 1 to 6 for the external disinfection of living beings.

8. Use of the preparation according to any one of Claims 1 to 6 for the disinfection of rooms and/or objects.

9. Use of the preparation according to any one of Claims 1 to 6 for killing bacterial spores.

## Revendications
### Revendications pour les Etats contractants suivants : DE, GB, FR, IT, NL, SE, LI, CH, BE, AT

1. Composition pharmaceutique contenant un solvant et de l'iode complexé par de la polyvinylpyrrolidone, caractérisée en ce que le solvant est un mélange d'éthanol et d'isopropanol en un rapport compris entre 2:1 et 1:2, la composition ne contenant pas plus de 6 % en poids d'eau.

2. Composition selon la revendication 1, caractérisée en ce que le solvant contient de l'éthanol et de l'isopropanol en un rapport de 1:1.

3. Composition selon la revendication 1 ou 2, caractérisée en ce que la composition ne contient pas plus de 2 % en poids, et en particulier contient entre 0 et 0,5 % en poids d'eau.

4. Composition selon l'une quelconque des revendications 1 à 3, caractérisée en ce que la composition contient de la polyvinylpyrrolidone en une quantité suffisante pour complexer totalement l'iode contenu.

5. Composition selon l'une quelconque des revendications 1 à 4, caractérisée en ce que la teneur en $I_2$ de la composition est comprise entre 0,05 et 2 % en poids.

6. Composition selon la revendication 5, caractérisée en ce que la teneur en $I_2$ est comprise entre 0,1 et 1 % en poids.

7. Utilisation de la composition selon l'une quelconque des revendications 1 à 6, pour la désinfection externe d'êtres humains.

8. Utilisation de la composition selon l'une quelconque des revendications 1 à 6, pour la désinfection de pièces et/ou d'objets.

9. Utilisation de la composition selon l'une quelconque des revenications 1 à 6, pour la dévitalisation de spores bactériennes.

**Revendications pour les Etats contractants suivants : ES, GR**

1. Procédé pour la préparation d'une composition pharmaceutique contenant un solvant et de l'iode complexé par de la polyvinylpyrrolidone, caractérisé en ce que l'on utilise comme solvant un mélange d'éthanol et d'isopropanol en un rapport compris entre 2:1 et 1:2, et la composition ne contient pas plus de 6 % en poids d'eau.

2. Procédé selon la revendication 1, caractérisé en ce que le solvant contient de l'éthanol et de l'isopropanol en un rapport de 1:1.

3. Procédé selon la revendication 1 ou 2, caractérisé en ce que la composition ne contient pas plus de 2 % en poids et en particulier contient entre 0 et 0,5 % en poids d'eau.

4. Procédé selon l'une quelconque des revendications 1 à 3, caractérisé en ce que la composition contient de la polyvinylpyrrolidone en une quantité suffisante pour complexer totalement l'iode contenu.

5. Procédé selon l'une quelconque des revendications 1 à 4, caractérisé en ce que la teneur en $I_2$ de la composition est comprise entre 0,05 et 2 % en poids.

6. Procédé selon la revendication 5, caractérisé en ce que la teneur en $I_2$ est comprise entre 0,1 et 1 % en poids.

7. Utilisation de la composition selon l'une quelconque des revendications 1 à 6, pour la désinfection externe d'êtres humains.

8. Utilisation de la composition selon l'une quelconque des revendications 1 à 6, pour la désinfection de pièces et/ou d'objets.

9. Utilisation de la composition selon l'une quelconque des revenications 1 à 6, pour la dévitalisation de spores bactériennes.

**Patentansprüche**
**Patentansprüche für folgende Vertragsstaaten : DE, GB, FR, IT, NL, SE, LI, CH, BE, AT**

1. Eine pharmazeutische Zubereitung, die ein Lösungsmittel und Polyvinylpyrrolidon-komplexiertes Jod enthält, dadurch gekennzeichnet, daß das Lösungsmittel eine Mischung aus Ethanol und Isopropanol im Verhältnis zwischen 2:1 und 1:2 ist, wobei die Zubereitung nicht mehr als 6 Gew.-% Wasser enthält.

2. Zubereitung nach Anspruch 1, dadurch gekennzeichnet, daß das Lösungsmittel Ethanol und Isopropanol im Verhältnis 1:1 enthält.

3. Zubereitung nach Anspruch 1 oder 2, dadurch gekennzeichnet, daß die Zubereitung nicht mehr als 2 Gew.-%, und insbesondere zwischen 0 und 0,5 Gew.-% Wasser enthält.

4. Zubereitung nach einem der Ansprüche 1 bis 3, dadurch gekennzeichnet, daß die Zubereitung Polyvinylpyrrolidon in einer zur vollständigen Komplexierung des $I_2$-Gehaltes ausreichenden Menge enthält.

5. Zubereitung nach einem der Ansprüche 1 bis 4, dadurch gekennzeichnet, daß der $I_2$-Gehalt der Zubereitung zwischen 0,05 und 2 Gew.-% liegt.

6. Zubereitung nach Anspruch 5, dadurch ge-

kennzeichnet, daß der $I_2$-Gehalt zwischen 0,1 und 1 Gew.-% liegt.

7. Verwendung der Zubereitung nach einem der Ansprüche 1 bis 6 zur äußerlichen Desinfektion von Lebewesen.

8. Verwendung der Zubereitung nach einem der Ansprüche 1 bis 6 zur Desinfektion von Räumen und/oder Gegenständen.

9. Verwendung der Zubereitung nach einem der Ansprüche 1 bis 6 zur Abtötung bakterieller Sporen.

**Patentansprüche für folgende Vertragsstaaten : ES, GR**

1. Verfahren zur Herstellung einer pharmazeutischen Zubereitung, die ein Lösungsmittel und Polyyvinylpyrrolidon-komplexiertes Jod enthält, dadurch gekennzeichnet, daß als Lösungsmittel eine Mischung aus Ethanol und Isopropanol im Verhältnis zwischen 2:1 und 1:2 verwendet wird und die Zubereitung nicht mehr als 6 Gew.-% Wasser enthält.

2. Verfahren nach Anspruch 1, dadurch gekennzeichnet, daß das Lösungsmittel Ethanol und Isopropanol im Verhältnis von 1:1 enthält.

3. Verfahren nach Anspruch 1 oder 2, dadurch gekennzeichnet, daß die Zubereitung nicht mehr als 2 Gew.-%, und insbesondere zwischen 0 und 0,5 Gew.-% Wasser enthält.

4. Verfahren nach einem der Ansprüche 1 bis 3, dadurch gekennzeichnet, daß die Zubereitung Polyvinylpyrrolidon in einer zur vollständigen Komplexierung des $I_2$-Gehaltes ausreichenden Menge enthält.

5. Verfahren nach einem der Ansprüche 1 bis 4, dadurch gekennzeichnet, daß der $I_2$-Gehalt der Zubereitung zwischen 0,05 und 2 Gew.-% liegt.

6. Verfahren nach Anspruch 5, dadurch gekennzeichnet, daß der $I_2$-Gehalt zwischen 0,1 und 1 Gew.-% liegt.

7. Verwendung der Zubereitung nach einem der Ansprüche 1 bis 6 zur äußerlichen Desinfektion von Lebewesen.

8. Verwendung der Zubereitung nach einem der Ansprüche 1 bis 6 zur Desinfektion von Räumen und/oder Gegenständen.

9. Verwendung der Zubereitung nach einem der Ansprüche 1 bis 6 zur Abtötung bakterieller Sporen.